Europäisches Patentamt

European Patent Office

Office européen des brevets

(19)

(11) **EP 1 296 731 B1**

(12) **FASCICULE DE BREVET EUROPEEN**

(45) Date de publication et mention
de la délivrance du brevet:
**07.09.2005 Bulletin 2005/36**

(21) Numéro de dépôt: **01947537.5**

(22) Date de dépôt: **20.06.2001**

(51) Int Cl.$^7$: **A61M 5/30**

(86) Numéro de dépôt international:
**PCT/FR2001/001922**

(87) Numéro de publication internationale:
**WO 2001/097884 (27.12.2001 Gazette 2001/52)**

(54) **SERINGUE SANS AIGUILLE MUNIE D'UN RESERVOIR MODULABLE**

NADELLOSE SPRITZE MIT EINEM ANPASSBAREN BEHÄLTER

NEEDLELESS SYRINGE PROVIDED WITH MODULAR RESERVOIR

(84) Etats contractants désignés:
**AT BE CH CY DE DK ES FI FR GB GR IE IT LI LU
MC NL PT SE TR**

(30) Priorité: **22.06.2000 FR 0007984**

(43) Date de publication de la demande:
**02.04.2003 Bulletin 2003/14**

(73) Titulaire: **Crossject
Cédex 04 (FR)**

(72) Inventeurs:
- **ALEXANDRE, Patrick
  F-70100 Gray (FR)**
- **BROUQUIERES, Bernard
  F-83100 Toulon (FR)**
- **ROLLER, Denis
  F-91590 La Ferte Alais (FR)**

(56) Documents cités:
**WO-A-00/35520      WO-A-00/48654
WO-A-95/03844      US-A- 2 764 977
US-A- 4 059 107      US-A- 4 124 024
US-A- 4 941 880**

## Description

Le domaine technique de l'invention est celui des seringues sans aiguille pré-remplies et jetables, fonctionnant avec un générateur de gaz, et utilisées pour les injections intradermiques, sous-cutanées et intramusculaires, de principe actif liquide à usage thérapeutique en médecine humaine ou vétérinaire. Une seringue selon le préambule est connue du document US-A-4 941 880.

Pour les dispositifs d'injection selon l'invention, un principe actif liquide est constitué par un liquide plus ou moins visqueux, ou un mélange de liquide, ou un gel. Le principe actif peut être un solide mis en solution dans un solvant approprié pour l'injection. Il peut également être représenté par un solide pulvérulent mis en suspension plus ou moins concentré dans un liquide approprié. La granulométrie du principe doit être compatible avec le diamètre des conduits pour éviter les bouchages.

Les seringues sans aiguille selon l'invention possèdent la particularité de pouvoir injecter une quantité variable de principe actif liquide par la seule modification de leur réservoir, toutes leurs autres caractéristiques étant intégralement conservées.

Par ce biais, il devient possible de concevoir facilement et à moindre coût des seringues sans aiguille sur étagère, munies de leur propre dose de principe actif liquide sans avoir à retoucher leur géométrie ni adapter le générateur de gaz à la dose de principe actif liquide à injecter.

Les seringues sans aiguille conçues pour injecter des quantités variables de principe actif liquide existent déjà et ont fait l'objet de plusieurs brevets.

On peut citer, par exemple, le brevet US 4,913,699 qui décrit un injecteur sans aiguille destiné à contenir une quantité variable de principe actif liquide. La pression du liquide en sortie d'injecteur est réglée par la différence de section entre le piston de poussée situé en amont, et un bouchon-piston de section réduite logé dans le canal terminal d'injection. Le brevet Wo 00/10630 se rapporte également à un injecteur sans aiguille présentant, entre autre, la particularité d'expulser une quantité variable de principe actif liquide en réduisant la course du piston de poussée. En effet, le réservoir de principe actif qui a une taille standard, est systématiquement rempli au maximum de sa capacité, et le piston, dont la course peut être réduite par un système d'évents de dépressurisation ne va assurer l'injection que d'une partie du principe actif enfermé dans le réservoir. Après l'injection, du principe actif liquide demeure dans le fond du réservoir dans les cas où la course du piston a été réduite.

Enfin, on peut évoquer le brevet 95/03844, relatif à un injecteur sans aiguille présentant également la caractéristique de pouvoir injecter une quantité variable de principe actif par réduction de la course du piston de poussée. Ici, le vissage d'un bouton occasionne le déplacement du piston en même temps que la compression d'un ressort. Lorsque le piston se retrouve à la position requise, le déclenchement de l'injecteur libère alors ledit piston qui est propulsé par le ressort qui se détend.

Les seringues sans aiguille selon l'invention permettent d'injecter une quantité variable de principe actif liquide par simple réajustement du volume disponible dans le réservoir. En effet, quelle que soit la quantité à injecter, le réservoir présente un encombrement constant au sein de la seringue et c'est seulement son volume interne qui est modifié, sans avoir à redéfinir, pour chaque cas, les caractéristiques du générateur de gaz ou du piston de poussée, et sans avoir à redimensionner les différents compartiments de la seringue. Les seringues sans aiguille selon l'invention contiennent ainsi la quantité exacte de principe actif à injecter et préservent leur pleine efficacité sans rien avoir à modifier et en évitant de conserver du principe actif en fond de réservoir après injection.

L'objet de la présente invention concerne une seringue sans aiguille comprenant un générateur de gaz, un réservoir constitué par un tube obturé par un bouchon-piston amont et un bouchon aval entre lesquels est contenu le principe actif liquide, et un dispositif d'injection, caractérisée en ce que ladite seringue est apte à injecter une quantité variable de principe actif en adaptant, par déplacement d'un bouchon-piston aval dans le tube, le volume compris entre les deux bouchons-pistons à la quantité de principe actif à injecter.

En effet, les seringues sans aiguille selon l'invention sont dimensionnées de manière à conserver leur capacité d'injection malgré une modification dimensionnelle de leur réservoir. Pour toute la suite du brevet, on appelle « colonne de liquide » l'ensemble constitué par les deux bouchons-pistons et le liquide de principe actif.

De façon avantageuse, le bouchon-piston aval est initialement entièrement inclus dans le tube, sans le dépasser.

De façon générale, les seringues sans aiguille selon l'invention sont destinées à injecter proprement du principe actif liquide à travers la peau sans provoquer de déperdition dudit principe sur la peau pour vitesse insuffisante. Le générateur de gaz est donc apte, dans une première phase, à communiquer une vitesse très élevée au principe actif liquide de façon à assurer à celui-ci un pouvoir de pénétration immédiat à travers la peau et, dans une deuxième phase, à maintenir, au moyen d'une pression à peu près constante, un niveau de vitesse suffisant pour le liquide, de façon à garantir son passage à travers la peau durant toute la durée de l'injection une fois la perforation assurée. La libération des gaz par le générateur va engendrer la création d'un espace sous pression entre ledit générateur et le bouchon-piston amont dû au déplacement dudit bouchon-piston. Cet espace sous pression dont le volume est croissant jusqu'à la fin de l'injection, correspond à une chambre d'expansion des gaz. De façon préférentielle, ladite chambre d'expansion n'existe pas lorsque la seringue ne fonctionne pas. Cependant, il est possible de concevoir une seringue sans aiguille selon l'invention avec un espace libre préexistant entre le générateur de gaz et le bouchon-piston de poussée. Typiquement, la variation de pression au cours du temps délivrée par le générateur de gaz dans l'espace situé entre ledit générateur et le bouchon-piston amont fait apparaître, dans un premier temps, un pic de pression instantanée et très intense, puis, dans un deuxième temps, l'instauration d'une pression à peu près constante toujours supérieure au niveau de pression seuil d'injection. Le terme « générateur de gaz » est un terme générique désignant sans distinction particulière une source énergétique qui, lorsqu'elle est activée par l'utilisateur, est apte à produire des gaz dans la seringue.

Préférentiellement, le générateur de gaz est un générateur de gaz pyrotechnique comprenant une charge pyrotechnique et un système d'initiation.

Avantageusement, le système d'initiation fait intervenir un dispositif de percussion et une amorce. Il est également possible d'utiliser un système d'initiation à base d'un cristal piézo-électrique ou d'un rugueux constitué par deux surfaces de frottement dont le déplacement créé une zone d'inflammation. Par rapport à une réserve de gaz sous pression, une charge pyrotechnique présente l'avantage d'être peu encombrante et évite à la seringue d'être soumise en permanence à une pression interne élevée, y compris en phase de stockage. De façon préférentielle, la charge pyrotechnique est constituée par le mélange d'une première poudre et d'une deuxième poudre, la première poudre ayant une vivacité dynamique supérieure à celle de la deuxième poudre. De façon avantageuse, la première poudre a une vivacité dynamique supérieure à 8 $(MPa.s)^{-1}$ et la deuxième poudre a une vivacité dynamique inférieure à 16 $(MPa.s)^{-1}$.

En effet, un moyen d'obtenir le profil de pression permettant d'injecter proprement le principe actif liquide, est de mettre en oeuvre une charge pyrotechnique constituée par le mélange d'une poudre vive et d'une poudre lente. Une poudre est qualifiée de « vive » si elle possède une vivacité dynamique élevée et de « lente » si elle possède une vivacité dynamique faible. Ainsi, la poudre vive permet de créer instantanément un pic de pression très intense tandis que la poudre lente assure, pour le reste de l'injection, un niveau de pression sensiblement constant et suffisamment élevé pour permettre le passage à travers la peau du principe actif liquide une fois la perforation réalisée. Ce type de mélange de poudre est tout à fait adapté aux seringues sans aiguille selon l'invention. Les valeurs de vivacité dynamique données ci-avant sont déterminées par la formule :

$$L(z) = \frac{1}{P} \cdot \frac{1}{Pmax} \left( \frac{dP}{dt} \right)$$

où P est la pression instantanée correspondant à l'état d'avancement z.

Pmax est la pression maximale atteinte. $\frac{dP}{dt}$ est la dérivée de la pression au cours du temps

$$z = \frac{P}{Pmax}.$$

Elles correspondent à des valeurs à mi-combustion, c'est à dire pour z = 0,5 réalisées en bombe manométrique dans les conditions suivantes :

- volume de chambre : 27,8 $cm^3$
- densité de chargement : 0,036 $g/cm^3$
- masse de poudre : 1 g

La poudre dite lente aura toujours une vivacité dynamique inférieure à celle de la poudre vive. Avantageusement, les deux poudres sont mélangées en vrac, c'est à dire que les deux poudres se présentent à l'état de grains qui sont mélangés aléatoirement, sans ordonnancement particulier, la poudre résultante épousant la forme du conteneur dans lequel elle se trouve, tout en ménageant des interstices entre les grains. Mais il peut également être envisagé qu'au moins l'une des deux poudres se présentent de façon ordonnée ou particulière, comme, par exemple, sous la forme d'un fagot de brins ou sous la forme d'un grain unique de taille importante, voire sous forme agglomérée.

Préférentiellement le volume du réservoir compris entre les deux bouchons-pistons peut varier de 0,1 ml à 2 ml suivant les besoins de l'injection.

De façon avantageuse, le bouchon-piston amont est situé, de façon fixe, à l'une des deux extrémités du tube. De façon préférentielle, au moins un piston est logé entre le générateur de gaz et le bouchon-piston amont. Préférentiellement, le bouchon-piston amont est en contact avec le piston.

Avantageusement le tube est en verre et a une épaisseur comprise entre 0,5 mm et 4 mm et préférentiellement entre 1,5 mm et 2,5 mm. Le tube en verre doit être suffisamment épais pour résister à une pression interne très élevée, sans subir de dommages irréversibles comme des fissurations ou même un éclatement.

De façon avantageuse, les deux bouchons-pistons sont réalisés en matériau déformable. Ils sont notamment obtenus par moulage d'élastomères compatibles avec le principe actif liquide sur une longue durée. Ces élastomères peuvent, par exemple, être des chlorobutyl ou des bromobutyl. De façon préférentielle le dispositif d'injection comporte au moins deux conduits périphériques d'injection qui sont situés à l'extérieur d'une pièce creuse servant de réceptacle au bouchon-piston aval, la profondeur de ladite pièce permettant le dégagement des entrées des conduits périphériques lorsque ledit bouchon-piston aval vient au contact du fond de ladite pièce creuse.

En terme de fonctionnement, la colonne de liquide se déplace jusqu'à ce que le bouchon-piston aval vienne occuper la pièce creuse. Une fois bloqué dans ladite pièce, le bouchon-piston se déforme légèrement de façon à dégager l'entrée des conduits latéraux d'injection et à permettre au principe actif d'être expulsé. Préférentiellement, la longueur du tube est constante. En effet, les seringues sans aiguille selon l'invention sont prévues pour recevoir un réservoir d'encombrement standard dicté par la longueur du tube, les variations de son volume interne situé entre les deux bouchons-pistons étant sans conséquence sur ses dimensions externes. Avantageusement, le piston est logé dans un corps creux de même diamètre interne que celui du tube et situé dans son prolongement, ledit corps creux et ledit tube étant maintenus dans une enveloppe.

De façon préférentielle, l'enveloppe est en matière plastique et exerce une légère compression sur le tube de façon à accroître sa résistance au cisaillement. De cette manière, le tube est apte à résister à de plus grandes pressions internes. Selon une variante de réalisation de l'invention, le réservoir comprenant le tube et les deux bouchons-pistons constitue une pièce autonome de ladite seringue. Autrement dit, le réservoir peut être introduit dans la seringue ou retiré à tout moment. De même, il peut être rempli à part, avec la dose exacte de principe actif, puis introduit ensuite dans la seringue.

De façon avantageuse, un espace libre existe entre le bouchon-piston aval et le fond de la pièce creuse. Ainsi, quelle que soit la quantité de principe actif à injecter et donc quelle que soit la position du bouchon-piston aval dans le tube, la colonne de liquide devra subir un déplacement notable, avant que l'injection à proprement parler ne débute. Plus la quantité de produit à injecter est faible, plus la position du bouchon-piston aval est voisine de celle du bouchon-piston amont et plus la course de la colonne de liquide, avant injection, est importante. L'existence d'un espace libre entre le bouchon-piston aval et le fond du réceptacle est particulièrement adapté aux seringues sans aiguille selon l'invention. En effet, dans l'hypothèse où l'évolution de la pression au cours du temps dans la chambre d'expansion est adaptée à une injection propre de principe actif liquide sans déperditions, il est impératif, pour les petites doses de principe actif à injecter, de ménager un espace libre suffisamment important entre le bouchon-piston aval et le fond du réceptacle pour permettre à la colonne de liquide d'être suffisamment accélérée avant le début de l'injection. Cette accélération est rendue nécessaire pour compenser la diminution de pression dans la chambre d'expansion due à l'accroissement important de son volume, induit par le déplacement de la colonne de liquide. Autrement dit, pour les petites quantités de principe actif à injecter, les gaz émis par le générateur de gaz vont permettre à la colonne de liquide de se déplacer pendant toute sa course avec une vitesse croissante. Donc, plus le déplacement de la colonne est important plus son énergie d'impact sur le fond du réceptacle est importante en raison de la vitesse croissante et ce, malgré la chute de pression significative dans la chambre d'expansion.

A titre d'exemple, la montée en pression dans le liquide pour un réservoir de 0,5 ml intervient plus tôt que pour un réservoir de 0,2 ml. Ceci s'explique par le fait que pour une quantité de 0,5 ml, la course de déplacement est plus faible que pour une quantité de 0,2 ml. L'égalité des intensités des pics de pression maximum pour les deux configurations correspondant à 0,2 ml et à 0,5 ml se justifie par le fait que l'énergie cinétique d'impact de la colonne de liquide compense une diminution de la poussée intrinsèque du liquide, en raison d'une baisse de la pression gazeuse due à un accroissement du volume de l'espace créé en aval de la charge pyrotechnique.

Les seringues sans aiguille selon l'invention permettent d'injecter des quantités de principe actif liquide par simple adaptation de leur réservoir, sans avoir à modifier ni leur géométrie, ni leur dimension ni même le générateur de gaz et sans avoir à rajouter d'autres pièces. Elles ne nécessitent donc aucun redimensionnement et aucun usinage supplémentaire, sources de coûts plus élevés.

En outre, elles demeurent d'un encombrement constant puisque le réservoir, quelle que soit la quantité de principe actif liquide à injecter, conserve ses dimensions externes.

Enfin, l'adaptation du réservoir reste une opération à la fois simple et précise, permettant d'avoir sur étagère des seringues sans aiguille transportant la quantité exacte de principe actif liquide à injecter.

On donne ci-après la description détaillée d'un mode de réalisation préféré de l'invention en se référant aux figures 1 à 4.

La figure 1 est une vue en coupe axiale longitudinale partielle d'une seringue sans aiguille n'ayant pas encore fonctionné.

La figure 2 est une vue en coupe axiale longitudinale partielle de la seringue de la figure 1 en cours de fonctionnement, l'injection n'ayant pas encore débuté.

La figure 3 est une vue en coupe axiale longitudinale partielle de la seringue de la figure 1 en fin de fonctionnement, l'injection étant terminée.

La figure 4 est un diagramme simplifié comparatif des variations de pression au cours du temps, d'abord dans l'espace libre créé en aval de la charge pyrotechnique, puis dans le liquide pour un réservoir de 0,5 ml de principe actif et enfin dans le liquide pour un réservoir de 0,2 ml de principe actif.

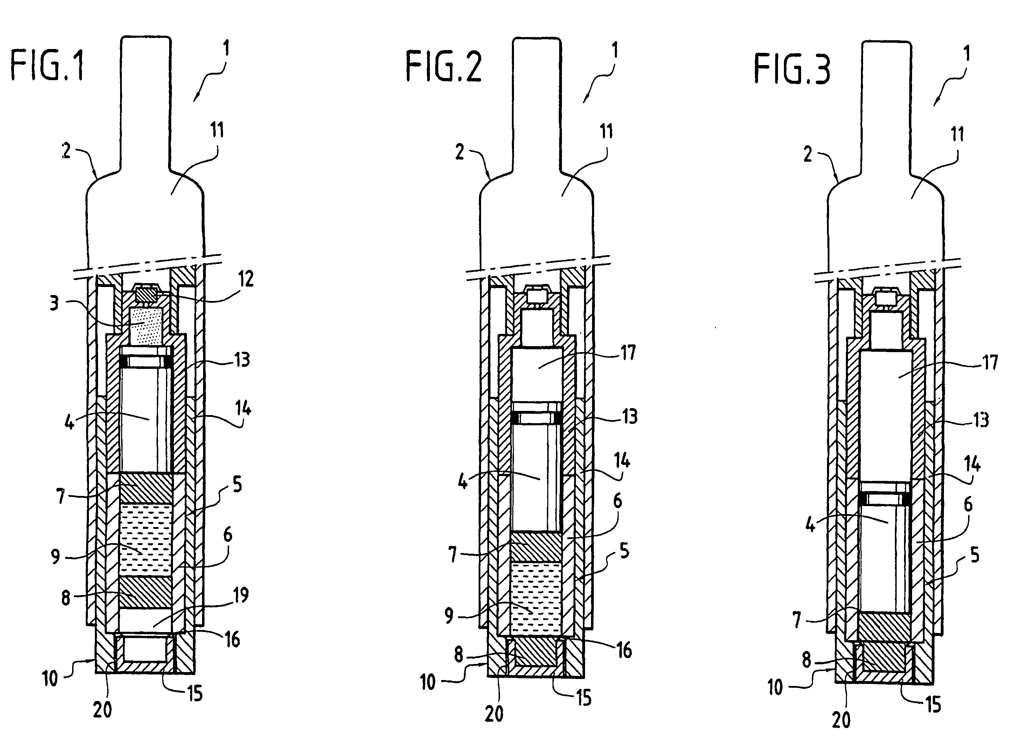

En se référant à la figure 1, une seringue sans aiguille 1 selon l'invention comprend un générateur pyrotechnique 2 de gaz composé d'un système d'initiation et d'une charge pyrotechnique 3, un piston de poussée 4, un réservoir 5 constitué par un tube 6 en verre obturé par un bouchon-piston amont 7 et un bouchon-piston aval 8 entre lesquels est contenu le principe actif liquide 9, et un dispositif d'injection 10.

Le système d'initiation fait intervenir un dispositif de percussion non représenté sur la figure 1 et une amorce 12. Le dispositif de percussion 11 qui est déclenché par un bouton-poussoir comprend un ressort et une masselotte munie d'un percuteur. La masselotte est bloquée par au moins une bille coincée entre ladite masselotte et le bouton-poussoir, et ledit bouton-poussoir possède une gorge interne circulaire.

La charge pyrotechnique 3 est composée par le mélange d'une poudre vive, comme par exemple une poudre poreuse à base de nitrocellulose et d'une poudre lente comme une poudre à base de nitrocellulose présentant des grains heptatubulaires. La charge pyrotechnique 3 débouche dans un corps creux 13 sensiblement cylindrique, lui-même prolongé par le tube 6 du réservoir 5, ledit tube 6 ayant le même diamètre interne que celui du corps creux 13. Le tube 6 est en continuité du corps creux 13 et à son contact, ces deux pièces 6, 13 ayant également le même diamètre externe. Celles-ci sont donc parfaitement alignées l'une sur l'autre et sont maintenues dans cette configuration par une enveloppe 14 en matière plastique exerçant après montage une légère compression sur le corps creux 13 ainsi que sur le tube 6. L'enveloppe 14 prend naissance environ à mi-longueur du corps creux 13 et se prolonge au delà du tube 6 en verre par une avancée cylindrique creuse formant un canal interne supplémentaire venant se rajouter au canal interne du tube 6, ledit canal supplémentaire ayant une section supérieure à celle du canal interne du tube 6 et approximativement égale à celle définie par le cercle fictif matérialisant la mi-épaisseur dudit tube 6.

Le canal supplémentaire permet de recevoir une pièce creuse 15 constituée par un corps cylindrique creux obturé à l'une de ses deux extrémités par une surface circulaire plane, ladite pièce 15 étant assimilable à un bouchon cylindrique creux. Ledit corps creux présente sur sa surface latérale externe une série de six rainures longitudinales, parallèles entre elles et régulièrement espacées.

La pièce 15 est emmanchée dans le canal supplémentaire de sorte que :

- la surface circulaire plane de ladite pièce 15 affleure l'extrémité libre de l'avancée de l'enveloppe 14,
- la paroi latérale externe du corps cylindrique creux de ladite pièce 15 est au contact de la paroi latérale interne du canal supplémentaire de sorte que les rainures longitudinales forment des conduits périphériques 20 d'injection,
- il subsiste un passage 16 entre l'extrémité libre du tube 6 et l'extrémité libre du corps cylindrique creux de ladite pièce 15.

Le corps cylindrique creux 13 dans lequel débouche la charge pyrotechnique 3 est entièrement occupé par le piston 4 de poussée. Le bouchon-piston amont 7 affleure l'une des deux extrémités du tube 6 en verre et ledit piston de poussée 4 est au contact à la fois de la charge pyrotechnique 3 et dudit bouchon-piston amont 7.

Le bouchon-piston aval 8 est situé en retrait de l'autre extrémité du tube 6 et le principe actif liquide 9 est enfermé dans l'espace dudit tube 6 délimité par les deux bouchons-pistons 7, 8. Les deux bouchons-pistons 7, 8 sont réalisés en matériau déformable à base d'élastomère. Le piston 4 de poussée est réalisé en matériau indéformable. En aval du bouchon-piston aval 8 existe un espace libre 19 constitué par le canal interne de la partie du tube 6 située en aval du bouchon-piston aval 8 et par le volume interne de la pièce 15 assimilable à un bouchon creux, ledit espace libre 19 étant en communication avec l'extérieur de la seringue 1 au moyen des six conduits périphériques 20 d'injection.

Le dispositif d'injection 10 comprend l'avancée de l'enveloppe 14 ainsi que la pièce creuse 15 emmanchée dans ladite avancée, et inclut donc l'espace libre 19 situé en aval du bouchon-piston aval 8 et les six conduits périphériques 20 d'injection.

Le mode de fonctionnement de ce mode de réalisation préféré de l'invention s'effectue comme suit.

L'utilisateur positionne la seringue 1 de façon à ce que son extrémité vienne en appui contre la peau du patient à traiter. Une pression sur le bouton poussoir permet de le faire coulisser le long de la seringue 1 jusqu'à ce que la gorge vienne au niveau de la bille qui bloque la masselotte. La bille en se dégageant dans la gorge libère la masselotte qui, sous l'effet du ressort qui se détend, est propulsée vers l'amorce 12, le percuteur en avant.

L'amorce 12 qui est alors initiée provoque la mise à feu de la charge pyrotechnique 3.

Sous l'effet de la combustion de la poudre vive, le piston 4 de poussée est brutalement déplacé dans le canal interne du corps creux 13 et du tube 6 provoquant le déplacement de la colonne de liquide. Une chambre d'expansion 17 des gaz se crée alors entre la charge pyrotechnique 3 et le piston 4 de poussée.

La figure 2 montre la seringue 1 en cours de fonctionnement, plus précisément à l'instant où la colonne de liquide vient en butée contre le fond du réceptacle formé par le volume interne de la pièce creuse 15 assimilable à un bouchon creux. La comparaison des figures 1 et 2 montre la distance parcourue par ladite colonne avec une vitesse croissante avant son impact. Le bouchon-piston aval 8 qui occupe intégralement le réceptacle s'écrase quelque peu, libérant alors le passage 16 servant d'entrée aux canaux 20 du dispositif d'injection 10, tandis que le bouchon-piston amont 7 se rapproche du bouchon-piston aval 8 piégé. Le principe actif liquide 9 est alors expulsé avec une pression sensiblement décroissante mais toujours supérieure à la pression seuil de diffusion du liquide après perforation de la peau, jusqu'à ce que les deux bouchons-pistons 7, 8 viennent au contact l'un de l'autre, comme le montre la figure 3.

Durant le déplacement du bouchon-piston amont 7, le volume de la chambre d'expansion 17 s'est continuellement accru. En fin d'injection, il ne reste plus de principe actif liquide dans le réservoir 5 de la seringue 1.

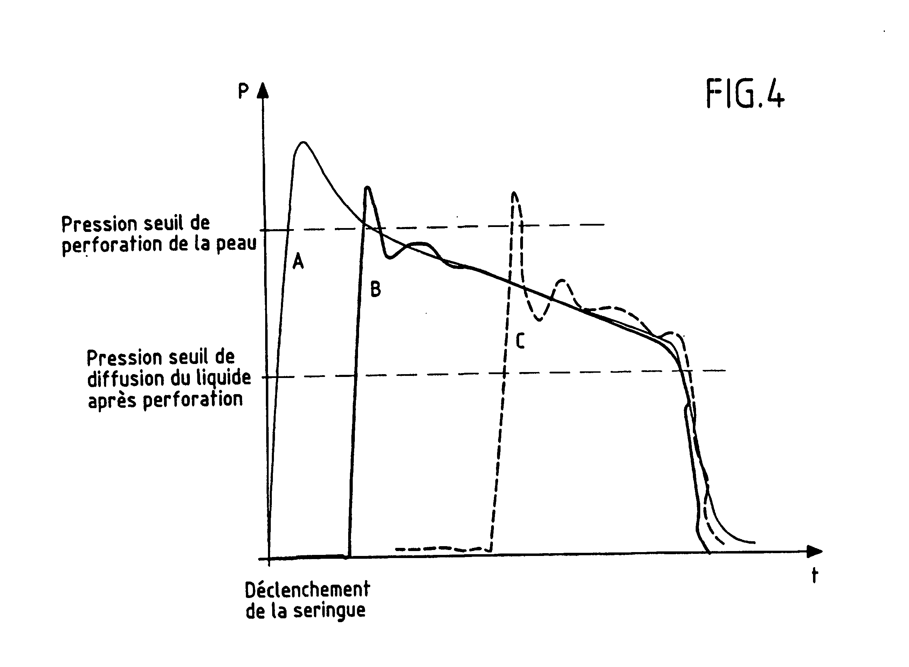

A titre d'exemple, en se référant à la courbe A du diagramme de la figure 4, la variation de pression au cours du temps induite par la combustion de la charge pyrotechnique 3 dans l'espace créé entre ladite charge 3 et le piston 4, fait apparaître instantanément un pic très intense suivi d'une phase de décroissance progressive et continue. L'apparition du pic très intense se réalise au bout d'environ 1 ms, la phase de décroissance dure, quant à elle, une vingtaine de millisecondes. La courbe B, qui illustre la variation de pression au cours du temps du principe actif liquide 9 en sortie de buse pour un réservoir 5 contenant initialement 0,5 ml, fait également apparaître un pic très intense suivi d'une phase de décroissance. Le pic se créé un certain temps après le déclenchement de la seringue, ce délai correspondant à la course de la colonne de liquide avant que le bouchon-piston aval 8 n'arrive en butée au fond de la pièce creuse 15.

Ce pic, qui demeure très intense, résulte de la conjonction de deux phénomènes :

- la poussée intrinsèque du liquide 9 par la pression gazeuse,
- le choc de la colonne de liquide au fond de la pièce creuse 15. En effet, ladite colonne se déplaçant avec une vitesse croissante dans le réservoir 5, le choc s'effectue en phase d'accélération, octroyant ainsi un surplus énergétique favorable à l'expulsion du liquide.

La phase de décroissance de la pression qui s'en suit est sensiblement alignée sur celle observée dans l'espace libre créé entre la charge pyrotechnique 3 et le piston 4.

Enfin, la courbe C qui représente la variation de pression au cours du temps du principe actif liquide 9 en sortie de buse pour un réservoir 5 contenant initialement 0,2 ml, montre un pic de pression très intense suivi d'une phase de décroissance continue. Le pic arrive plus tardivement que celui de la courbe B puisque la course de la colonne de liquide est plus importante. Les pics des courbes B et C ont à peu près la même intensité, car la poussée intrinsèque du liquide 9 par la pression gazeuse qui est inférieure dans le cas du réservoir contenant 0,2 ml en raison d'une baisse de pression gazeuse due à un accroissement plus important du volume de l'espace situé entre la charge pyrotechnique 3 et le piston 4, est compensée par une augmentation de l'énergie cinétique d'impact induite par un déplacement plus important de la colonne de liquide à vitesse croissante.

Le diagramme comparatif de la figure 4, montre que par simple déplacement du piston-bouchon aval 8, la seringue sans aiguille 1 selon l'invention conserve sa pleine efficacité en terme, d'abord, de perforation de la peau, puis, en terme de diffusion du liquide 9.

## Revendications

1. Seringue sans aiguille (1) comprenant un générateur de gaz (2), un réservoir (5) constitué par un tube (6) obturé par un bouchon-piston amont (7) et un bouchon aval (8) entre lesquels est contenu le principe actif liquide (9), ledit réservoir (6) pouvant avoir un volume variable par déplacement du bouchon-piston aval (8) dans ledit tube (6), et un dispositif d'injection (10), **caractérisé en ce que** le réservoir (5) comprenant le tube (6) et les deux bouchons-pistons (7,8) constitue une pièce autonome et détachable de ladite seringue (1) et le générateur de gaz (2) est un générateur pyrotechnique possédant une charge pyrotechnique (3) et un système d'initiation.

2. Seringue sans aiguille selon la revendication 1, **caractérisée en ce que** le bouchon-piston aval (8) est initialement entièrement inclus dans le tube (6).

3. Seringue sans aiguille selon la revendication 1, **caractérisée en ce que** la charge pyrotechnique (3) est constituée par le mélange d'une première poudre et d'une deuxième poudre, la première poudre ayant une vivacité dynamique supérieure à celle de la deuxième poudre.

4. Seringue sans aiguille selon la revendication 3, **caractérisée en ce que** la première poudre a une vivacité dynamique supérieure à 8 $(MPa.s)^{-1}$ et la deuxième poudre une vivacité dynamique inférieure à 16 $(MPa.s)^{-1}$.

5. Seringue sans aiguille selon la revendication 1, **caractérisée en ce que** le volume du réservoir (5) compris entre les deux bouchons-pistons (7, 8) peut varier de 0,1 ml à 2 ml.

6. Seringue sans aiguille selon la revendication 1, **caractérisée en ce que** le bouchon-piston amont (7) est situé, de façon fixe, à l'une des deux extrémités du tube (6).

7. Seringue sans aiguille selon la revendication 6, **caractérisée en ce qu'**au moins un piston (4) est logé entre le générateur de gaz (2) et le bouchon-piston amont (7).

8. Seringue sans aiguille selon la revendication 7, **caractérisée en ce que** le bouchon-piston amont (7) est en contact avec le piston (4).

9. Seringue sans aiguille selon la revendication 1, **caractérisée en ce que** le tube (6) est en verre et a une épaisseur comprise entre 0,5 mm et 4 mm.

10. Seringue sans aiguille selon la revendication 1, **caractérisée en ce que** les deux bouchons-pistons (7, 8) sont réalisés en matériau déformable.

11. Seringue sans aiguille selon la revendication 1, **caractérisée en ce que** le dispositif d'injection (10) comporte au moins deux conduits périphériques (20) d'injection qui sont situés à l'extérieur d'une pièce creuse (15) servant de réceptacle au bouchon-piston aval (8), la profondeur de ladite pièce (15) permettant le dégagement des entrées des conduits périphériques (20) lorsque ledit bouchon-piston aval (8) vient au contact du fond de ladite pièce creuse (15).

12. Seringue sans aiguille selon l'une quelconque des revendications 1 ou 9, **caractérisée en ce que** la longueur du tube (6) est constante.

13. Seringue sans aiguille selon la revendication 7, **caractérisée en ce que** le piston (4) est logé dans un corps creux (13) de même diamètre interne que celui du tube (6) et situé dans son prolongement, ledit corps creux (13) et ledit tube (6) étant maintenus dans une enveloppe (14).

14. Seringue sans aiguille selon la revendication 11, **caractérisée en ce qu'**un espace libre (19) existe entre le bouchon-piston aval (8) et le fond de la pièce creuse (15).


**Patentansprüche**

1. Nadellose Spritze (1), die einen Gasgenerator (2), ein Reservoir (5), das aus einem Rohr (6) besteht, das von einem vorderen Stopfen-Kolben (7) und einem hinteren Stopfen (8) verschlossen wird, zwischen denen der flüssige Wirkstoff (9) enthalten ist, wobei das Reservoir (6) durch Verschiebung des hinteren Stopfen-Kolbens (8) im Rohr (6) ein variables Volumen haben kann, und eine Injektionsvorrichtung (10) aufweist, **dadurch gekennzeichnet, dass** das das Rohr (6) und die beiden Stopfen-Kolben (7, 8) aufweisende Reservoir (5) ein autonomes und von der Spritze (1) lösbares Bauteil bildet, und dass der Gasgenerator (2) ein pyrotechnischer Generator ist, der eine pyrotechnische Ladung (3) und ein Zündsystem besitzt.

2. Nadellose Spritze nach Anspruch 1, **dadurch gekennzeichnet, dass** der hinter Stopfen-Kolben (8) ursprünglich vollständig im Rohr (6) enthalten ist.

3. Nadellose Spritze nach Anspruch 1, **dadurch gekennzeichnet, dass** die pyrotechnische Ladung (3) aus der Mischung eines ersten Pulvers und eines zweiten Pulvers besteht, wobei das erste Pulver eine größere dynamische Geschwindigkeit hat als das zweite Pulver.

4. Nadellose Spritze nach Anspruch 3, **dadurch gekennzeichnet, dass** das erste Pulver eine dynamische Geschwindigkeit von mehr als 8 $(MPa.s)^{-1}$ und das zweite Pulver eine dynamische Geschwindigkeit von weniger als 16 $(MPa.s)^{-1}$ hat.

5. Nadellose Spritze nach Anspruch 1, **dadurch gekennzeichnet, dass** das zwischen den beiden Stopfen-Kolben (7, 8) liegende Volumen des Reservoirs (5) von 0,1 ml bis 2 ml variieren kann.

6. Nadellose Spritze nach Anspruch 1, **dadurch gekennzeichnet, dass** der vordere Stopfen-Kolben (7) ortsfest an einem der beiden Enden des Rohrs (6) angeordnet ist.

7. Nadellose Spritze nach Anspruch 6, **dadurch gekennzeichnet, dass** mindestens ein Kolben (4) zwischen dem Gasgenerator (2) und dem vorderen Stopfen-Kolben (7) angeordnet ist.

8. Nadellose Spritze nach Anspruch 7, **dadurch gekennzeichnet, dass** der vordere Stopfen-Kolben (7) mit dem Kolben (4) in Kontakt steht.

9. Nadellose Spritze nach Anspruch 1, **dadurch gekennzeichnet, dass** das Rohr (6) aus Glas ist und eine Dicke zwischen 0,5 mm und 4 mm aufweist.

10. Nadellose Spritze nach Anspruch 1, **dadurch gekennzeichnet, dass** die beiden Stopfen-Kolben (7, 8) aus verformbarem Material bestehen.

11. Nadellose Spritze nach Anspruch 1, **dadurch gekennzeichnet, dass** die Injektionsvorrichtung (10) mindestens zwei Umfangsinjektionskanäle (20) aufweist, die sich außerhalb eines hohlen Bauteils (15) befinden, das als Aufnahmebehälter für den hinteren Stopfen-Kolben (8) dient, wobei die Tiefe des Bauteils (15) das Freisetzen der Eingänge der Umfangskanäle (20) ermöglicht, wenn der hintere Stopfen-Kolben (8) mit dem Boden des hohlen Bauteils (15) in Kontakt kommt.

12. Nadellose Spritze nach einem der Ansprüche 1 oder 9, **dadurch gekennzeichnet, dass** die Länge des Rohrs (6) konstant ist.

13. Nadellose Spritze nach Anspruch 7, **dadurch gekennzeichnet, dass** der Kolben (4) in einem Hohlkörper (13) mit gleichem Innendurchmesser wie das Rohr (6) angeordnet ist, der sich in dessen Verlängerung befindet, wobei der Hohlkörper (13) und das Rohr (6) in einer Hülle (14) gehalten werden.

14. Nadellose Spritze nach Anspruch 11, **dadurch gekennzeichnet, dass** ein Freiraum (19) zwischen dem hinteren Stopfen-Kolben (8) und dem Boden des hohlen Bauteils (15) vorhanden ist.

**Claims**

1. Needleless syringe (1) comprising a gas generator (2), a reservoir (5) consisting of a tube (6) closed by an upstream stopper-piston (7) and a downstream stopper (8) between which the liquid active principle (9) is contained, it being possible for the said reservoir (6) to have a variable volume by moving the downstream stopper-piston (8) in the said tube (6), and an injection device (10), **characterized in that** the reservoir (5) comprising the tube (6) and the two stopper-pistons (7,8) constitutes a self-contained detachable part of the said syringe (1) and the gas generator (2) is a pyrotechnic generator possessing a pyrotechnic charge (3) and an initiating system.

2. Needleless syringe according to claim 1, **characterized in that** the downstream stopper-piston (8) is initially entirely enclosed in the tube (6).

3. Needleless syringe, according to claim 1, **characterized in that** the pyrotechnic charge (3) consists of a mixture of a first powder and a second powder, the first powder having a dynamic vivacity greater than that of the second powder.

4. Needleless syringe according to claim 3, **characterized in that** the first powder has a dynamic vivacity greater than 8 $(Mpa.s)^{-1}$ and the second powder has a dynamic vivacity less than 16 $(Mpa.s)^{-1}$.

5. Needleless syringe according to claim 1, **characterized in that** the volume of the reservoir (5) enclosed between the two stopper-pistons (7,8) can vary from 0.1 ml to 2 ml.

6. Needleless syringe according to claim 1, **characterized in that** the upstream stopper-piston (7) is situated, in a fixed manner, at one of the two ends of the tube (6).

7. Needleless syringe according to claim 6, **characterized in that** at least one piston (4) is housed between the gas generator (2) and the upstream stopper-piston (7).

8. Needleless syringe according to claim 7, **characterized in that** the upstream stopper-piston (7) is in contact with the piston (4).

9. Needleless syringe according to claim 1, **characterized in that** the tube (6) is made of glass and has a thickness of between 0.5 mm and 4 mm.

10. Needleless syringe according to claim 1, **characterized in that** the two stopper-pistons (7,8) are made of a deformable material.

11. Needleless syringe according to claim 1, **characterized in that** the injection device (10) comprises at least two peripheral injection ducts (20) that are situated outside a hollow part (15) serving as a receptacle for the downstream stopper-piston (8), the depth of the said part (15) enabling the inlets of the peripheral ducts (20) to be disengaged when the said downstream stopper-piston (8) comes into contact with the bottom of the said hollow part (15).

12. Needleless syringe according to any one of claims 1 to 9, **characterized in that** the length of the tube (6) is constant.

13. Needleless syringe according to claim 7, **characterized in that** the piston (4) is housed in a hollow body (13) having the same internal diameter as that of the tube (6) and situated in its extension, the said hollow body (13) and the said tube (6) being held in an envelope (14).

14. Needleless syringe according to claim 11, **characterized in that** a free space (19) exists between the downstream stopper-piston (8) and the bottom of the hollow part (15).

FIG.1

FIG.2

FIG.3

EP 1 296 731 B1

FIG.4

P

Pression seuil de
perforation de la peau

Pression seuil de
diffusion du liquide
après perforation

A

B

C

Déclenchement
de la seringue

t

EP 1 296 731 B1